## Europäisches Patentamt

(19) **European Patent Office**

**Office européen des brevets**

(11) Publication number: **0 078 500**
**B1**

(12) **EUROPEAN PATENT SPECIFICATION**

(45) Date of publication of patent specification: **28.08.85**

(21) Application number: **82109930.6**

(22) Date of filing: **27.10.82**

(51) Int. Cl.⁴: **C 12 M 1/36,** C 12 N 1/00, C 12 N 1/18

(54) Method and apparatus for controlling the aerobic cultivation of yeasts.

(30) Priority: **04.11.81 JP 177527/81**

(43) Date of publication of application:
**11.05.83 Bulletin 83/19**

(45) Publication of the grant of the patent:
**28.08.85 Bulletin 85/35**

(84) Designated Contracting States:
**DE FR GB**

(56) References cited:
**CS-A- 181 337
DE-B-1 174 733
DE-C- 739 021
FR-A-2 293 488
FR-A-2 483 458
US-A-3 002 894
US-A-4 167 450**

**CHEMICAL ABSTRACTS, vol. 92, no. 13, March 1980, page 512, no. 109144a, Columbus Ohio (USA);
Allgemeine Mikrobiologie - H.G. Schlegel, 5th Ed. 1981, pp. 192,193,250,251,261,262**

(73) Proprietor: **Hitachi, Ltd.
5-1, Marunouchi 1-chome
Chiyoda-ku Tokyo 100 (JP)**

(72) Inventor: **Shimizu, Norio
2374-24, Ohkubo-cho
Hitachi-shi Ibaraki-ken (JP)**
Inventor: **Ueno, Masao
2-3-4-505, Imafuku-higashi Johtoh-ku
Ohsaka-shi Ohsaka-fu (JP)**
Inventor: **Odawara, Yooji
4-21-6, Nishinarusawa-cho
Hitachi-shi Ibaraki-ken (JP)**
Inventor: **Nishiumi, Masaharu
1166, Higashi-toyoi
Kadumatsu-shi Yamaguchi-ken (JP)**
Inventor: **Fujimoto, Masakatsu
1166, Higashi-toyoi
Kadumatsu-shi Yamaguchi-ken (JP)**
Inventor: **Matsushita, Nobuo
428-1, Higashi-toyoi
Kadumatsu-shi Yamaguchi-ken (JP)**

(74) Representative: **Patentanwälte Beetz sen. -
Beetz jun. Timpe - Siegfried - Schmitt-Fumian
Steinsdorfstrasse 10
D-8000 München 22 (DE)**

Courier Press, Leamington Spa, England.

## Description

The invention relates to a method and an apparatus for controlling the aerobic cultivation of yeasts.

In the production of yeasts e.g. for foodstuffs and livestock feed and baker's yeast, a cultivation process has been employed in which small amounts of a substrate are sequentially fed to the medium. In this process it is essential that the substrate fed to the medium is completely consumed by the yeast and the formation of by-products such as ethanol is suppressed.

No method has yet been established for the quick determination of the concentration and activity of yeasts during the cultivation. For this reason, a substrate feed system which feeds the substrate to the fermentation tank in accordance with a predetermined program has been used. However, this system can not feed the substrate in accordance with the activity of the yeast inside the fermentation tank and hence, has low productivity.

The present invention is directed to a method and an apparatus for improving the production efficiency of the yeast cultivation by suitably controlling the substrate feeding rate during the cultivation.

This object is accomplished according to the characterising clauses of claims 1 and 6. The sub-claims refer to preferred embodiments.

The substrate feeding method of the present invention is characterised in that the formation of by-products during cultivation is detected using a specific feeding rate $\alpha$ of the substrate as a parameter, and increasing the specific feeding rate $\alpha$ at a predetermined rate when no by-product is detected, and decreasing it when by-products are detected. Preferably, the specific feeding rate $\alpha$ is decreased at a rate greater than the predetermined increasing rate.

In the following, the invention is described with reference to the drawings.

Figures 1 through 6 are diagrams showing the relation between the $\alpha$ value and the ethanol formation;

Figures 7 and 8 are schematic views, each showing an example of the incubation apparatus of the present invention;

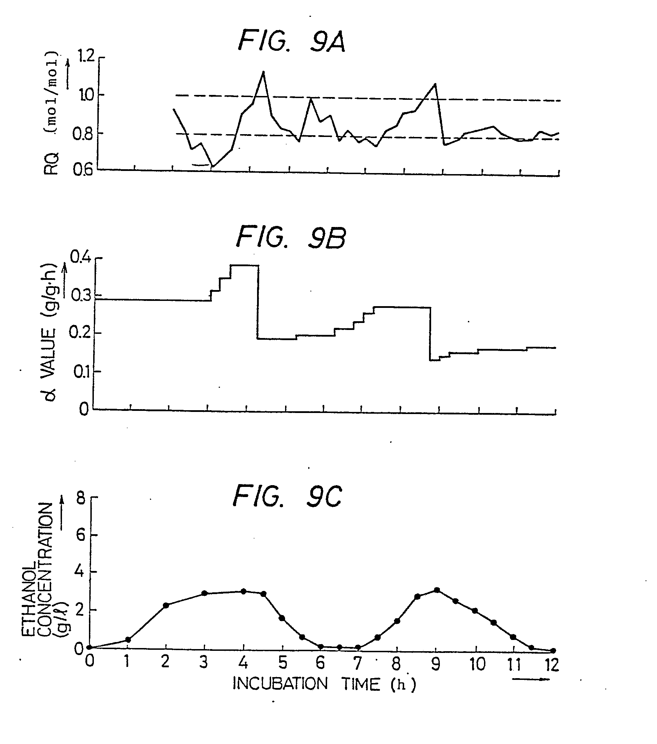

Figure 9 shows diagrams for explaining the cultivation in accordance with the present invention; and

Figure 10 is a diagram showing an example of the simulation of the yeast quantity in accordance with the present invention.

The substrate feeding method according to the invention will be described in more detail.

The specific feeding rate $\alpha$ is defined as

$$\alpha = \frac{F \cdot c_s}{V_c \cdot c_y} \qquad (1)$$

where F is the substrate feeding rate (l/h), $c_s$ is the substrate concentration (g/l), $V_c$ is the volume of the culture solution (l) and $c_y$ is the concentration of yeast (g/l).

Based on formula (1), the substrate feeding rate F can be expressed as

$$F = \alpha \cdot \frac{V_c \cdot c_y}{c_s} \qquad (2)$$

The $\alpha$ value, i.e. the substrate feeding rate per unit quantity of yeast, depends on the activity of the utilized enzymes. In the case of baker's yeast, the yeast can be multiplied by the substrate ingested by raising the $\alpha$ value and increasing the substrate feeding rate if the activity is high. If the activity is low, on the other hand, the ingested substrate glucose is converted into ethanol and $CO_2$, which lowers the productivity accordingly.

The activity of the enzymes means the capacity of the enzymes to completely consume the substrate fed and to produce the yeast mass without forming by-products such as ethanol. Although it was desired to employ a method for feeding the substrate which avoids the formation of by-products, no method was hitherto available for directly measuring this activity. The enzyme activity can be estimated on the basis of the oxygen consumption rate, however, the oxygen consumption rate changes with the substrate feeding speed. For this reason, this method alone is not sufficient.

According to the present invention an initial value of $\alpha$ is set at the start of the incubation, whereby the substrate is fed at a feeding rate corresponding to the set $\alpha$ value, and the $\alpha$ value is increased when no by-products are formed. In this case, the increasing rate of $\alpha$ is very critical: As a result of intensive investigation, it has been found that the $\alpha$ value should be increased by up to 20% and preferably 10%, in dependence of the change of the physiological enzyme activity.

The upper limit of the increase of the $\alpha$ value is 20%, because the feed of substrate becomes excessive if the increase is more than 20%. If the increase is too small, on the other hand, the substrate feed becomes insufficient, and the productivity of the yeast production cannot be improved. Hence, the preferred increasing rate is set at 10%.

If by-products are formed, the substrate feeding speed must be lowered, otherwise the by-products are formed continuously and the productivity drops. However, the formation of the by-products cannot be stopped if the $\alpha$ value is decreased at the same rate as the increase. This is because the mechanism of the multiplication of the yeast mass by decomposition of the substrate is different from the mechanism of the by-product formation by fermentation. It is assumed that once the enzymatic activity related to the formation of by-products becomes high, the flow of metabolic products is directed to the formation of the by-products. Accordingly, the decrease of the substrate feed becomes important. Corresponding investigations have revealed that the formation of the by-products is stopped and the by-products formed in the culture medium can be used again by the enzymes if the decreasing rate of the α value is at least 30% and preferably 50%.

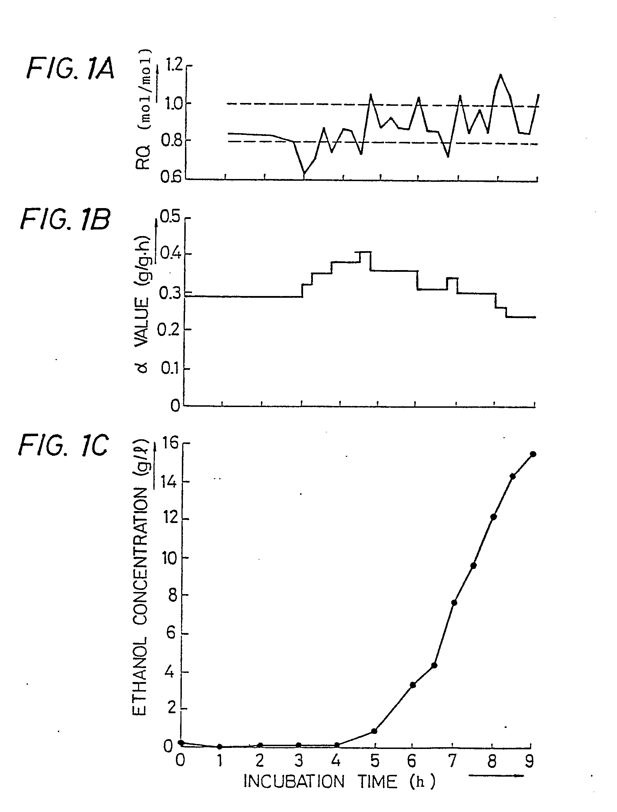

Figures 1, 2 and 3 illustrate examples of experiments of various substrate feeding systems using baker's yeast. In these examples, feedback control by the respiratory quotient (RQ) was started from 1 or 2 hours after the start of incubation and the α value was changed every 15 minutes so as to control the substrate feeding speed.

Figure 1 illustrates the behaviour of a system in which the α value was increased at a rate of 10% and was decreased at a rate of 10%. When the control range of RQ was from 0,8 to 1,0 mol/mol, RQ exceeded 1,0 mol/mol when ethanol was formed in an amount of about 1 g/l (Fig. 1A). From this point the α value was reduced at a rate of 10% but the formation of ethanol was not stopped and about 15,4 g/l of ethanol were formed. It is assumed that once ethanol is thus formed (Fig. 1C), the yeast cells reach a physiological fermentation a state which is suitable for the ethanol formation and the physiological conditions cannot be rapidly changed to a state suitable for the multiplication of the yeast cells if the decreasing rate of the α value is too small (Fig. 1B).

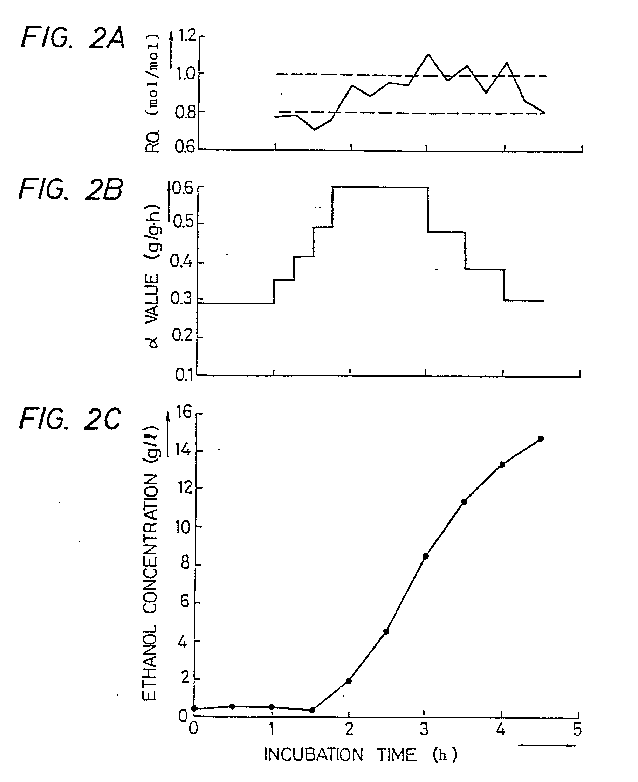

Figure 2 illustrates the situation that the α value was increased at a rate of 20% and was decreased at a rate of 20% (Fig. 2B). When the control range of RQ was from 0,8 to 1,0 mol/mol, RQ exceeded 1,0 mol/mol when 8,4 g/l of ethanol were formed (Figs. 1A and 1C) It is assumed that ethanol was formed in a greater amount than in the case of Figure 1 because the increasing rate of the α value was greater. Though the α value was thereafter reduced at a rate of 20%, the ethanol formation was not stopped and 14,7 g/l of ethanol were formed.

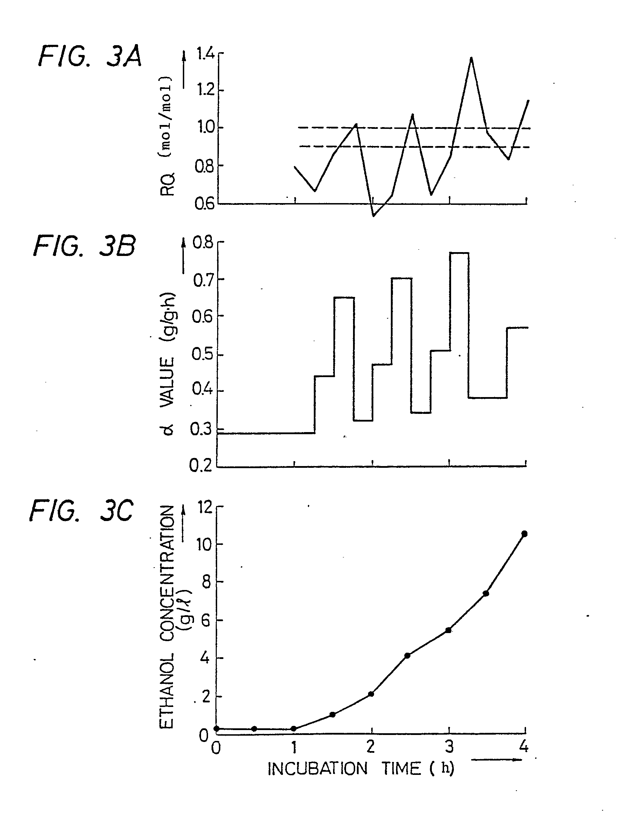

Figure 3 illustrates the case in which the α value was increased at a rate of 50% and was decreased at a rate of 50% (Fig. 3B). When the control range of RQ was from 0,9 to 1,0 mol/mol, RQ exceeded 1,0 mol/mol at the point when about 1,5 g/l of ethanol was formed (Figs. 3A and 3C). Though the α value was repeatedly increased and decreased, the ethanol formation was not stopped and 10,5 g/l of ethanol were formed. In addition, RQ caused hunching.

It has been found from the experiments described above that the increasing rate of the α value must be up to 20% because if it is 50%, RQ causes hunching. However, the increasing rate of 10% is most suitable because the quantity of ethanol formed is great at an increase by 20%. On the other hand, the ethanol formation cannot be inhibited if the decreasing rate of the α value is below 20%.

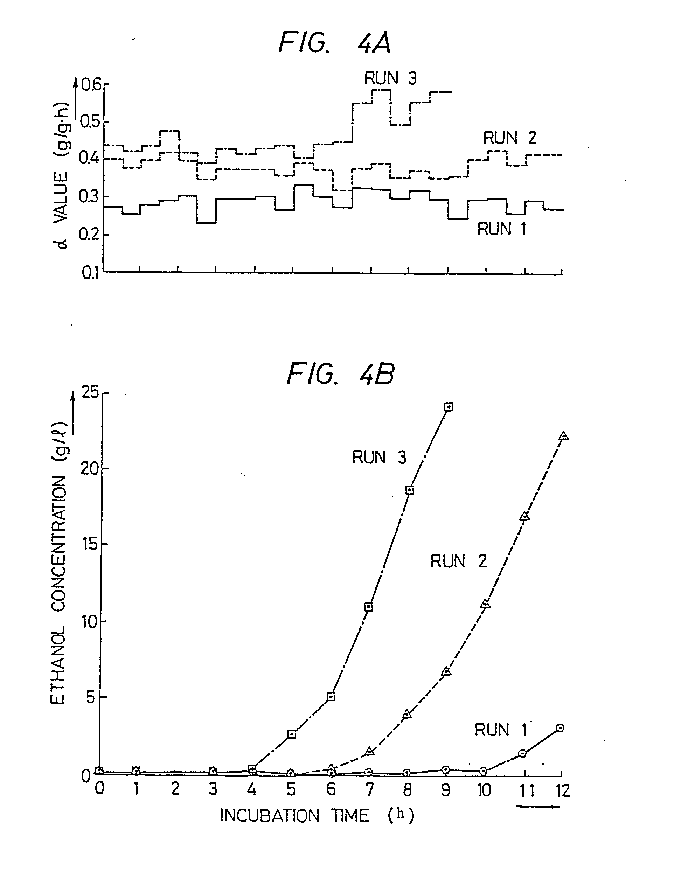

In Figures 1 and 2, ethanol was formed even if the α value was not changed. Figure 4 shows the result of an experiment carried out to confirm this phenomenon. When the substrate was fed at a substantially constant rate, i.e., 0,3 g/g·h in RUN 1, 0,39 g/g·h in RUN 2 and 0,42 g/g·h in RUN 3 (Fig. 4A), the ethanol formation occurred after 5 hours. 7 hours and 11 hours, respectively, in the order of higher α values (Fig. 4B). This means that the physiological activity of the yeast cells changes even if substrate feeding is effected with a constant α value. It is therefore assumed that the reason why the ethanol formation could not be stopped by merely resetting the α value to the original α value before ethanol was formed is that a change has occurred in the physiological activity of the yeast cells, that is, the ethanol formation has a time lag.

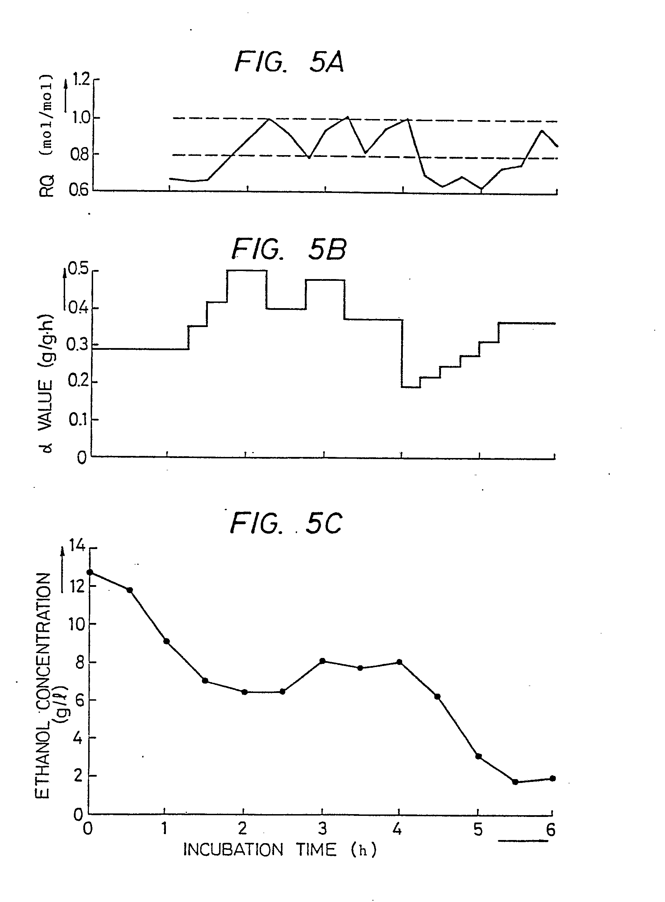

Next, Figure 5 illustrates the result of investigation of the decreasing rate of the α value. The incubation was started under a condition that 12,7 g/l of ethanol were present at the initial stage of incubation (Fig. 5C). In this case, too, the ethanol content could not be reduced effectively, though the α value was reduced at a rate of 20% at the point when RQ exceeded 1,0 mol/mol (Figs. 5A and 5B). When the α value was decreased at a rate of 50% 4 hours after the start of incubation, the ethanol concentration was drastically reduced, and could be reduced to 2 g/l, though the α value was thereafter increased at a rate of 20%. It can be seen from the above that if the decreasing rate of the α value is 20%, the rise of the ethanol concentration in the culture solution can be prevented but the ethanol cannot be reduced. However, if the decreasing rate is 50%, the physiological activity of the yeast cells can be changed so that the ethanol formation can be checked and ethanol consumption takes place. It has thus been found that the decreasing rate of the α value must be at least 30% and is preferably 50%.

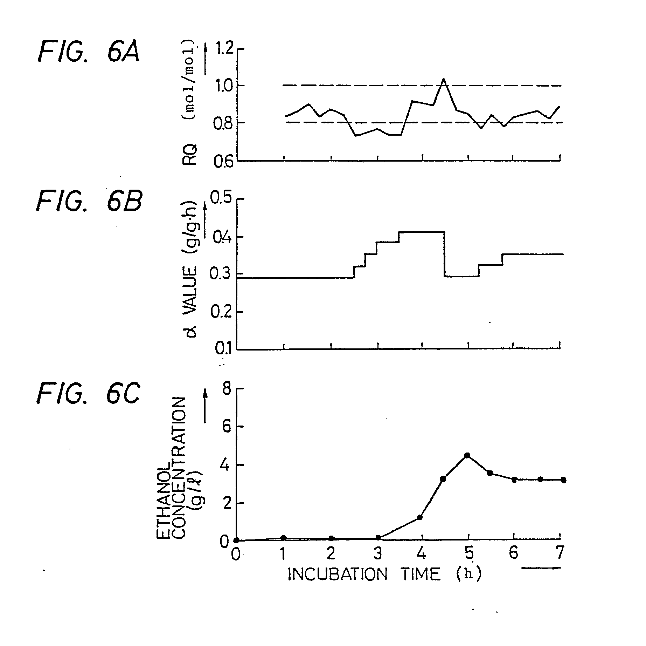

Figure 6 illustrates data when the α value was increased at a rate of 10% and was decreased at a rate of 30% (Fig. 6B). It can be understood that though the effect can be observed at a decreasing rate of 30%, it is considerably inferior to the decreasing rate of 50%.

As described in the foregoing it has been found that it is effective to increase the α value at a rate of up to 20% and preferably, 10% and reduces the α value at a rate of at least 30% and preferably 50% when by-products are formed, in order to change the physiological state of the yeast cells from the formation of the by-products to the multiplication.

To practise the present invention, the quantity of the yeast mass inside the incubation tank must be measured. Accordingly, the inventors of the present invention have found that the quantity of the yeast mass can be reasonably estimated from (1) the oxygen input and output, (2) the carbon input and output and (3) the multiplication model.

The estimation of the quantity of the yeast mass by the oxygen input and output can be based on the following formula:

$$\Delta c_y = (a_2 \cdot \Delta s - \Delta O_s - a_3 \cdot \Delta P)a_1 \qquad (3),$$

where $\Delta c_y$ is the increase of the yeast mass (g), $\Delta s$

is the amount of fed substrate (g), $O_2$ is the oxygen consumption (mol), P is the amount of by-products (g), $a_1$ is the oxygen amount required for complete combustion of the yeast cell mass (mol/g), $a_2$ is the oxygen amount required for complete combustion of the substrate (mol/g) and $a_3$ is the oxygen amount required for complete combustion of the by-products (mol/g) (for Saccharomyces cerevisiae and the substrate of example 1 $a_1=0,042$ mol/g and $a_2=0,033$ mol/g).

If the by-product formation is neglected as being small, an estimation of the quantity of the yeast cell mass in accordance with the formula (3) becomes possible because the amount of oxygen necessary for the combustion of the substrate is known and the oxygen consumption of the yeast cell mass can be obtained from the difference of the oxygen amount at the inlet and outlet.

The calculation of the quantity of the yeast cell mass from the carbon input and output is based on the following formula:

$$\Delta c_y = (b_2 \cdot \Delta s - 12 \cdot \Delta CO_2 - b_3 \cdot \Delta P)/b_1 \quad (4),$$

where $\Delta CO_2$ is the amount of carbon dioxide formed (mol), $b_1$ is the carbon content of the yeast cell mass (g/g), $b_2$ is the carbon content of the substrate (g/g) and $b_3$ is the carbon content of the by-products (g/g) (for Saccharomyces cerevisiae and the substrate of example 1 $b_1=0,47$ g/g and $b_2=0,40$ g/g). If the by-product formation is neglected as being small, an estimation of the quantity of the yeast cell mass in accordance with the formula (4) becomes possible because the carbon content of the substrate is known and the amount of carbon dioxide gas of the exhaust gas can be measured.

The calculation of the quantity of the yeast cell mass from the multiplication model is based on the following formula:

$$\frac{d(V_c \cdot c_y)}{dt} = \mu V_c \cdot c_y \quad (5),$$

$$\mu = \mu_{max} \cdot \frac{c_s}{K_s + c_s} \quad (6),$$

$$\frac{d(V_c \cdot c_s)}{dt} = F \cdot c_s - \frac{1}{Y_g} \mu V_c \cdot c_y - m V_c \cdot c_y \quad (7),$$

$$\frac{dV_c}{dt} = F \quad (8),$$

where $c_s$ is the substrate concentration in the solution (g/l), $\mu$ is the specific multiplication speed $(h^{-1})$, $\mu_{max}$ is the maximum specific multiplication speed $(h^{-1})$, $Y_g$ is the yield constant of the yeast cell mass with respect to the substrate (g/g), m is a maintenance constant with respect to the substrate $(g/g \cdot h)$, $K_s$ is the saturation constant (g/l) and t is the time (h).

The quantity of the yeast cell mass can be calculated by substituting the initial solution quantity $V_{co}$, the initial yeast concentration $c_{yo}$ and the coefficients $\mu_{max}$, $K_s$, $Y_g$ and m the measured value of the substrate feeding rate F in this formula. For Saccharomyces cerevisiae and the substrate of example 1 these coefficients are as follows: $\mu_{max}=0,37$ $h^{-1}$; $K_s=0,0648$ g/l; $y_g=0,52$ g/g; m=0,025 $g/g \cdot h$; $c_{so}=500$ g/l; $V_{co}=15$ l and $C_{yo}=50$ g/l.

As described above, the quantity of the yeast cell mass can be estimated from the oxygen input and output, the carbon input and output and the multiplication model. By combining these methods, the estimation of the quantity of the yeast cell mass can be made more reliable in practice.

To realize the optimal substrate feeding rate, the formation of by-products must be detected. It has been found that it can be detected satisfactorily by various methods such as by using the respiratory quotient (RQ) which is the ratio $Q_{CO_2}/Q_{O_2}$, by using the flow ratio of the outlet gas to the inlet gas (hereinafter called the "β value") and by a method which measures the volatile by-products in the exhaust gas such as ethanol by gas chromatrography.

In the case of using sugar as the substrate, RQ=1,0 represents the complete aerobic multiplication of the yeast cells RQ>1,0 represents the ethanol formation because the amount of carbon dioxide gas is great and RQ<1,0 represents that the substrate quantity is insufficient or the yeast cells consume resulting ethanol.

Accordingly, the α value is reduced when RQ exceeds 1,0, assuming that ethanol is formed, and the α value is raised when RQ is considerably below 1,0, assuming that the substrate concentration becomes insufficient.

The reason why the ethanol formation can be detected from the ratio of the outlet gas quantity to the inlet gas quantity (β value) is that when ethanol is formed, the outlet gas quantity becomes greater than the inlet gas quantity because $CO_2$ is formed in a larger amount. This method is extremely effective because it does not need the measurement of $O_2$ and $CO_2$ concentration that is necessary in the method using RQ.

The method of measuring the volatile components in the outlet gas directly measure the by-products such as ethanol. Since the by-products discharged into the culture solution are present in the exhaust gas, they can be measured by gas chromatography or semiconductor sensors.

Examples of the substrate to be used in the present invention include glucose, fructose, sucrose and molasses as industrial raw material. Auxiliary materials are those which are generally used for incubation, such as ammonium sulfate, urea, aqueous ammonia, potassium phosphate, yeast extract, magnesium sulfate, ferrous sulfate and various kinds of vitamines and minerals.

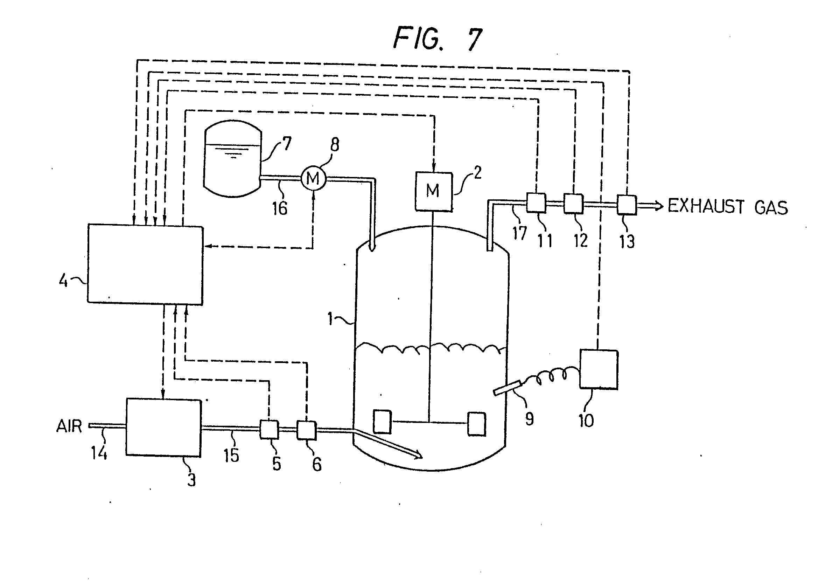

Figure 7 illustrates an example of the apparatus for practising the method of the present invention. A yeast seed is placed in an incubation tank 1 while the substrate is fed from a substrate tank 7 by a substrate feeding pump 8. In this case, data from an inlet oxygen partial pressure gauge 5, an inlet gas quantity meter 6, an outlet gas oxygen partial pressure gauge 11, a carbon dioxide partial pressure gauge 12 and an exhaust gas meter 13 are applied to a control computer 4 for calculating the quantity of the yeast mass in the incubation tank 1 from the multiplication model calculation, on the basis of the oxygen input and output and the carbon input and output and the substrate feeding rate. The substrate feeding rate is determined from this result and from the $\alpha$ value and the substrate feeding pump 8 is operated in accordance with the rate thus calculated. On the other hand, the RQ value and the ratio of the outlet gas quantity to the inlet gas quantity ($\beta$ value) are determined and the $\alpha$ value is changed in accordance with these values so as to control the substrate feeding rate. During incubation, the dissolved oxygen concentration must be kept at least 2 mg/l and a signal of a dissolved oxygen sensor 9 is sent to a dissolved oxygen meter 10. The value of the dissolved oxygen meter 10 is then applied to the control computer 4 so as to control the number of revolutions of a stirrer 2 and the inlet gas oxygen concentration and the inlet gas quantity by an oxygen separator 3.

In this manner, the dissolved oxygen concentration is kept at a constant value.

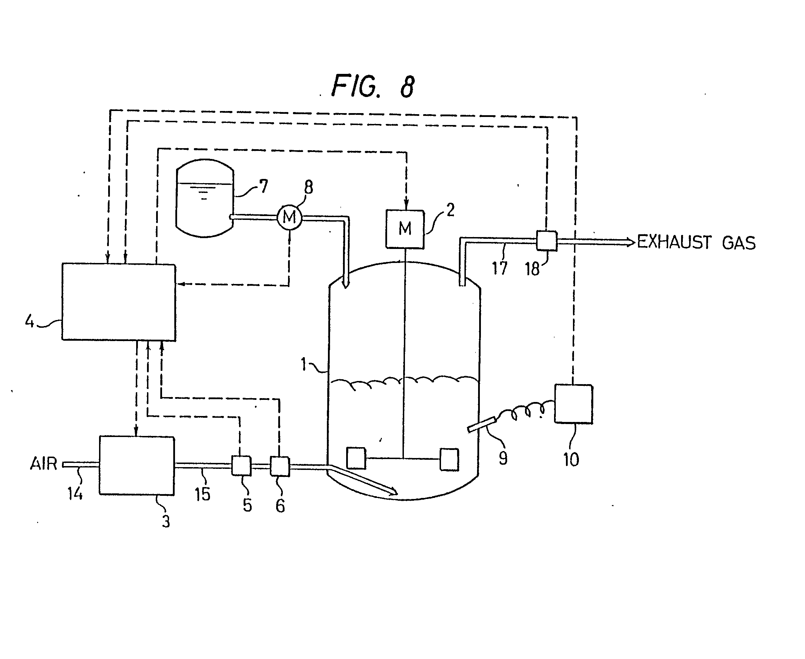

Figure 8 illustrates another example of the incubation apparatus of the present invention. This apparatus controls the substrate feed rate by means of a volatile component meter 18 disposed in the exhaust gas line in order to increase the $\alpha$ value when no volatile components as the by-products are detected, and to decrease the $\alpha$ value when they are detected. The construction of this apparatus is similar to that of the apparatus shown in Figure 7 with the exception of the exhaust gas line.

Next, the present invention will be explained more definitely with reference to examples.

Example 1

Strain: Saccharomyces cerevisiae (baker's yeast).

Medium: 500 g of glucose, 53,75 g of urea, 25 g of $Na_2HPO_4 \cdot 2H_2O$, 9,5 g of $MgSO_4 \cdot 7H_2O$, 5,5 g of KCl 62,5 g of sodium citrate, 12,2 g of yeast extract, 25 ml of a vitamine solution and 25 ml of a mineral solution were dissolved in 1 l of tap water and the pH was adjusted to 5,0.

The vitamine solution was prepared by dissolving 0,04 g of biotin, 0,08 g of vitame $B_1$, 2.0 g of vitamine $B_6$, 1,0 g of calcium pantothenate and 20 g of inositol in 1 l of distilled water. The mineral solution was prepared by dissolving 0,05 g of $CuSO_4 \cdot 5H_2O$, 0,8 g of $ZnSO_4 \cdot 7H_2O$ and 0,3 g of $FeSO_4 (NH_4)_2 \cdot 6H_sO$ in 1 l of distilled water.

Incubating conditions:

50 l jar fermenter; 30° C; pH 5,0. The dissolved oxygen concentration was adjusted within the range of 4 to 6 mg/l by the number of revolutions of a stirrer, the oxygen partial pressure and the quantity of the feed gas. The oxygen partial pressure of the feed gas was changed by use of an air compressor in combination with an oxygen cylinder. The internal pressure of the incubation tank was adjusted to 0,5 kg/cm$^2$ and the carbon dioxide concentration of the exhaust gas was adjusted within 20%. The initial solution quantity was 15 l, the initial yeast cell concentration was 50 g/l and the $\alpha$ value was set to 0,3 g/g · h. The average of the yeast cell quantities obtained from the oxygen input and output and from the carbon input and output was used as the yeast cell quantity inside the incubation tank. In this case, $a_1 - 0,042$, $a_2 = 0,033$, $b_1 = 0,47$ and $b_2 = 0,40$ were used as the coefficients. The formation of ethanol as by-product was detected by the RQ value. The $\alpha$ value was increased at a rate of 10% when RQ < 0,8 mol/mol and was decreased at a rate of 50% when RQ > 1,0 mol/mol.

Results:

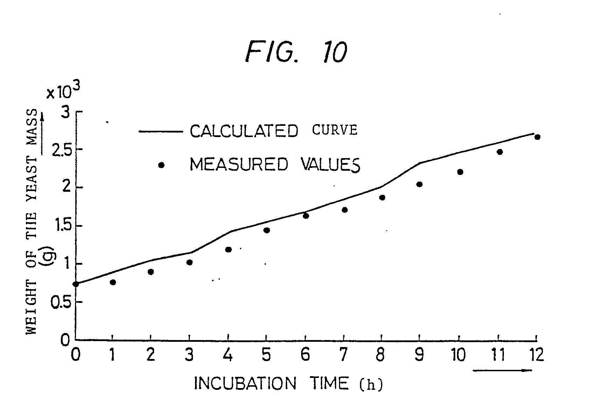

The ethanol concentration could be kept below 3,4 g/l throughout the 12 hours' incubation period as shown in Figure 8. The calculated value of the yeast cell mass quantity was substantially in agreement with the measured value as shown in Figure 10. Thus, the yeast cell mass concentration reached a high concentration of 104 g/l and its yield (ratio of multiplied yeast cell mass to fed glucose) was 0,43 g/g. The final culture solution quantity was 25,5 l.

Example 2:

Strain: Saccharomyces cerevisiae (baker's yeast).

Medium: Same as in Example 1.

Incubating conditions:

The procedure of Example 1 were followed in the same way except that the determination of the yeast cell mass quantity inside the incubation tank was carried out with use of the multiplication model $\mu_{max} = 0,37$ l/h; $K_s = 0,0648$ g/l, $Y_g = 0,52$ g/g and m = 0,025 g/g were used as the coefficients of the multiplication model.

Results:

The ethanol concentration could be kept within the range of 0,15 to 4,2 g/l throughout the incubation period of 10 hours. The yeast cell mass concentration reached a high level of 100 g/l and its yield was 0,43 g/g. The final culture solution quantity was 24,3 l.

Example 3

Strain: Saccharomyces cerevisiae (baker's yeast).

Medium: Same as in Example 1.

Incubating conditions:

The ethanol formation was detected from the ratio of the outlet gas quantity to the inlet gas quantity (β value) and the α value was increased at a rate of 10% when β<0,95 was decreased at a rate of 50% when β<1,0. The other conditions were the same as in Example 1.

Results:
The ethanol concentration could be kept below 1 g/l throughout the incubation period of 10 hours. The yeast cell mass concentration reached a high level of 105 g/l and its yield was 0,44 g/g. The final culture solution quantity was 24,1 l.

Example 4
Strain: Saccharomyces cerevisiae (baker's yeast).
Medium: Same as in Example 1.

Incubating conditions:
The exhaust gas was introduced into a gas chromatograph to detect the ethanol formation. The α value was increased at a rate of 10% when no ethanol was formed and was decreased at a rate of 50% when the ethanol concentration in the exhaust gas exceeded 1 ppm. The other conditions were the same as in Example 2.

Results:
The ethanol concentration could be kept below 1,4 g/l throughout the incubation period of 12 hours. The yeast cell mass concentration reached a high level of 106 g/l and its yield was 0,45 g/g. The final culture solution quantity was 24,4 l.
Since according to the present invention the substrate feeding can be controlled in such a manner that the formation of by-products is reduced, yeast cell mass concentrations in the culture medium of more than 100 g/l can be accomplished and hence the productivity of the process can be improved.

**Claims**

1. A method for controlling the aerobic cultivation of yeasts by adjusting the feed of the substrate to an incubation tank, whereby by-products can be formed in dependence of the substrate feed, characterised by

— feeding the substrate at an initial feeding rate corresponding to a predetermined initial value of the specific feeding rate α being defined as

$$\alpha = \frac{F \cdot c_s}{V_c \cdot c_y}$$

wherein
F=the substrate feeding rate (l/h)
$c_s$=the substrate concentration (g/l)
$V_c$=the volume of the culture solution (l) and
$c_y$=the concentration of yeast (g/l)

— with detection of the formation of by-products and
— increasing the specific feeding rate α of the substrate by up to 20% when no by-product is detected and
— decreasing the specific feeding rate α of the substrate by at least 30% when by-products are detected.

2. A method according to claim 1, characterised in that the specific feeding rate α is increased by up to 10% when no by-product is detected and is decreased by 50% when by-products are detected.

3. A method according to claim 1 or 2, characterised in that the weight of the yeast mass in the incubation tank is calculated from the oxygen input and output.

4. A method according to one of claims 1 or 2, characterised in that the weight of the yeast mass in the incubation tank is calculated from the carbon input and output.

5. A method according to claim 1 or 2, characterised in that the weight of the yeast mass in the incubation tank is calculated from a multiplication model based upon the weight of fed substrate.

6. An apparatus for controlling the aerobic cultivation of yeasts in an incubation tank by adjusting the substrate feed rate according to one of claims 1 to 5, characterised by

— a gas flow meter (6) measuring the velocity of the inlet gas,
— oxygen and carbon dioxide gas analyzers (5, 11, 12, 13) measuring the oxygen and carbon dioxide gas concentrations and partial pressures in the inlet and outlet gases, respectively,
— a substrate feeder (7) whose substrate feeding rate can be controlled on the basis of a computer output signal and
— a computer (4) performing calculations using the input signals from said gas flow meter (6) and from said oxygen and carbon dioxide gas analyzers (5, 11, 12, 13) and applying the results of calculations as an output signal to said substrate feeder (7).

**Patentansprüche**

1. Verfahren zur Kontrolle der aeroben Kultivierung von Hefen durch Einstellen der Substratzufuhr zu einem Inkubations-gefäß, wobei Nebenprodukte in Abhängigkeit der Substratzufuhr gebildet werden können, gekennzeichnet durch

— eine Substratzufuhr mit einer Angangsgeschwindigkeit, die einem vorbestimmten Anfangswert der spezifischen Zufuhrgeschwindigkeit α entspricht, die wie folgt definiert ist:

$$\alpha = \frac{F \cdot c_s}{V_c \cdot c_y}$$

wobei:
F=die Substratzufuhrgeschwindigkeit (l/h)
$c_s$=die Substratkonzentration (g/l)
$V_c$=das Volumen der Kulturlösung (l) und
$c_y$=die Konzentration der Hefe (g/l)

— Nachweis der gebildeten Nebenprodukte und
— die Erhöhung der spezifischen Substratzufuhr-geschwindigkeit α bis zu 20%, wenn kein Nebenprodukt nachweisbar ist, und
— die Verminderung dez spezifischen Substratzufuhrgeschwindigkeit α um mindestens 30%, wenn Nebenprodukte nachweisebar sind.

2. Verfahren nach Anspruch 1, dadurch gekennzeichnet, daß die spezifische Substratzufuhrgeschwindigkeit α bis zu 10% erhöht wird, wenn kein Nebenprodukt nachweisbar ist, und um 50% vermindert wird, wenn Nebenprodukte nachgewiesen werden.

3. Verfahren nach Anspruch 1 oder 2, dadurch gekennzeichnet, daß das Gewicht der Hefemasse im Inkubationsgefäß anhand der Sauerstoffzufuhr und—abgabe berechnet wird.

4. Verfahren nach Anspruch 1 oder 2, dadurch gekennzeichnet, daß das Gewicht der Hefemasse im Inkubationsgefäß anhand der Kohlenstoffzufuhr und—abgabe berechnet wird.

5. Verfahren nach Anspruch 1 oder 2, dadurch gekennzeichnet, daß das Gewicht der Hefemass im Inkubationsgefäß anhand eines Multiplikationsmodells auf der Basis des Gewichts der Substratzufuhr berechnet wird.

6. Vorrichtung zur Kontrolle der aeroben Kultivierung von Hefen in einem Inkubationsgefäß durch Einstellen der Substratzufuhrgeschwindigkeit gemäß einem der Ansprüche 1 bis 5, gekennzeichnet durch

— einen Gasdurchflußzähler (6) zur Bestimmung der Gaszufuhr,
— Sauerstoff- und Kohlendioxidanalysatoren (5, 11, 12, 13), zur Bestimmung der Sauerstoff- und Kohlendioxidkonzentrationen und -partialdrücke in den zugeführten bzw abgezogenen Gasen,
— eine Substratzufuhrvorrichtung (7), deren Substratzufuhrgeschwindigkeit aufgrund eines Rechner-Auslaßsignals kontrolliert werden kann, und
— einen Rechner (4), der mit den Einlaßsignalen des Gasdurchflußzähler (6) und der Sauerstoff- und Kohlendioxidanalysatoren (5, 11, 12, 13) rechnet und die Ergebnisse der Berechnungen als Auslaßsignal an der Substratzufuhrvorrichtung (7) anwendet.

**Revendications**

1. Procédé pour contrôler la culture aérobie de levures au moyen du réglage de l'alimentation du substrat dans une cuve d'incubation, ce qui a pour effet que des sous-produits peuvent être formés en fonction de l'alimentation en substrat, caractérisé par

— l'alimentation en substrat s'effectue à un débit d'alimentation initiale correspondant à une valeur initiale prédéterminée du débit spécifique d'alimentation définie par

$$\alpha = \frac{F \cdot c_s}{V_c \cdot c_y}$$

dans lequel
F=débit d'alimentation du substrat (l/h)
$c_s$=concentration du substrat (g/l)
$V_c$=volume de la solution de culture (l), et
$c_y$=concentration de la levure (g/l)

— avec détection de la formation de sous-produits, et
— accroissement du débit spécifique α du substrat, jusqu'à 20% lorsqu'aucun sous-produit n'est détecté, et
— réduction du débit spécifique d'alimentation α du substrat, d'au moins 30% lorsque des sous-produits sont détectés.

2. Procédé selon la revendication 1, caractérisé en ce que le débit d'alimentation spécifique α est accru jusqu'à une valeur de 10% lorsqu'aucun sous-produit n'est détecté et est réduit de 50% lorsque des sous-produits sont détectés.

3. Procédé selon la revendication 1 ou 2, caractérisé en ce que le poids de la masse de levure dans la cuve d'incubation est calculé à partir de l'entrée et de la sortie de l'oxygène.

4. Procédé selon l'une des revendications 1 ou 2, caractérisé en ce que le poids de la masse de levure dans la cuve d'incubation est calculé à partir de l'entrée et de la sortie de carbone.

5. Procédé selon la revendication 1 ou 2, caractérisé en ce que le poids de la masse de levure dans la cuve d'incubation est calculé à partir d'un modèle de multiplication, basé sur le poids du substrat introduit.

6. Appareil pour contrôler la culture aérobie de levures dans une cuve d'incubation moyennant le réglage de dèbit d'alimentation en substrat selon l'une des revendications 1 à 5, caractérisé par

— un débimètre à gaz (6) mesurant la vitesse du gaz introduit,
— des analyseurs de gaz pour l'oxygène et le gaz carbonique (5, 11, 12, 13) mesurant les teneurs de gaz en oxygène et en gaz carbonique et les pressions partielles de gaz d'entrée et de sortie respectivement,
— un dispositif (7) d'alimentation du substrat, dont le débit d'alimentation du substrat peut être commandé sur la base du signal de sortie d'un calculateur, et
— un calculateur (4) effectuant les calculs en utilisant les signaux d'entrée délivrés par ledit débimètre à gaz (6) et délivrés par lesdits analyseurs de gaz (5, 11, 12, 13) pour l'oxygène et le gaz carbonique, et envoyant les résultats des calculs en tant que signal de sortie audit dispositif (7) d'alimentation du substrat.

FIG. 1A

FIG. 1B

FIG. 1C

FIG. 2A

FIG. 2B

FIG. 2C

INCUBATION TIME (h)

FIG. 3A

FIG. 3B

FIG. 3C

INCUBATION TIME ( h)

## FIG. 4A

## FIG. 4B

## FIG. 5A

## FIG. 5B

## FIG. 5C

INCUBATION TIME (h)

FIG. 6A

FIG. 6B

FIG. 6C

INCUBATION TIME (h)

FIG. 7

EXHAUST GAS

AIR

0 078 500

# FIG. 8

EXHAUST GAS

AIR

FIG. 9A

FIG. 9B

FIG. 9C

INCUBATION TIME (h)

# FIG. 10